# EUROPÄISCHE PATENTSCHRIFT

(11) **EP 1 053 756 B1**
(45) Veröffentlichungstag und Bekanntmachung des Hinweises auf die Patenterteilung: **22.01.2003**
(21) Anmeldenummer: 99109832.8
(22) Anmeldetag: 19.05.1999
(51) Int. Cl.: A61L 24/10

(54) **Blutstillende Zubereitung**
Haemostatic composition
Composition hémostatique

(43) Veröffentlichungstag der Anmeldung: 22.11.2000
(73) Patentinhaber: Resorba Chirurgisches Nahtmaterial Franz Hiltner GmbH & Co., 90443 Nürnberg (DE)
(72) Erfinder: Hiltner, Claus Martin, 90408 Nürnberg (DE)
(74) Vertreter: Schneck, Herbert, Dipl.-Phys., Dr.

(56) Entgegenhaltungen:
- EP-A- 0 090 997
- US-A- 4 606 337

## Beschreibung

Die Erfindung richtet sich auf eine blutstillende Zubereitung umfassend die Komponenten Kollagen, Fibrinogen und Thrombin.

Im Rahmen der chirurgischen Blutstillung werden seit langem Kollagenprodukte als Filme, Lyophilisate sowie in Form feiner Granulate oder Pulver eingesetzt. Kollagen als natürliches, resorbierbares Stützprotein mit geringem allergischen Potential hat sich dabei als ökonomisches und sicheres Hilfsmittel erwiesen.

Seit mehreren Jahrzehnten haben sich daneben verschiedene Produkte etabliert, deren hämostatische Wirkung durch die Reaktion auf die Wundfläche aufgebrachter Blutproteine (aus Blutseren gewonnen) herbeigeführt wird.

Diese sogenannten Gewebekleber funktionieren nach dem Prinzip der Polymerisation von Fibrinogen mit Thrombin zu einem Polymerisat des fadenbildenden Fibrins und simulieren damit den natürlichen Gerinnungsvorgang.

Diese Art von Gewebekleber wird in der Chirurgie häufig zur Verbindung von Weichteilgeweben, wie z. B. in der Leber- und Milzchirurgie, oder auch zur regenerativen Fixierung von Sehnen, wie z. B. der Achillessehne, verwendet.

Gemeinsam ist diesen Anwendungsprozeßen, daß die Komponenten Fibrinogen einerseits und Thrombin andererseits in getrennten Spritzenkolben verwahrt und angereicht werden. Die Vereinigung geschieht erst unmittelbar vor der Applikation über eine gemeinsame Mischkanüle.

Aufgrund der flüssigen Darreichung der Proteinkomponenten ist es notwendig, diese zur Stabilisierung und Haltbarmachung tiefgefroren aufzubewahren. Dies stellt erhebliche logistische Anforderungen an den Anwender dieser Produkte.

Für den Arzt ist dieses Produkt schnell und unkompliziert einzusetzen. Daneben erspart die Lagerfähigkeit bei Raumtemperatur erhebliche logistische Zusatzbelastungen.

Im Rahmen minimal invasiver, chirurgischer Eingriffe werden Fibrinkleber, Kollagene und deren Kombination zur Umgehung z.T. umständlicher intracorporaler Naht- und Klammertechniken eingesetzt.

Fibrinkleber werden dazu über doppellumige Kapillarschläuche und Kollagene zum Teil über spezielle Applikationshilfen in den Körper eingebracht. Im Allgemeinen erfordern diese Einsatzgebiete von den Chirurgen eine große Geschicklichkeit und Erfahrung.

Seit kurzem wird auch berichtet, im Rahmen der Wunderstversorgung sowie zum Abdichten eröffnete Blutgefäße (z.B. nach Herzkathederuntersuchungen) Fibrinogen und Thrombin in gefriergetrockneter Form zur Blutstillung direkt auf die zu versorgende Wundfläche aufzusprühen. Nachteil des reinen Fibrinogen/Thrombineinsatzes ist die geringe Saugfähigkeit für Körperflüssigkeiten, der ein wichtiger Beitrag zur blutstillenden Wirkung zugeschrieben wird. Daneben ist es aufgrund des Keimkontaktes mit Wundflüssigkeiten entstehenden gelartigen Fibrinkomplexes nicht möglich, größere Mengen zur Steigerung der Blutstillung einzusetzen.

In einer schon seit langem bekannten Variante wird ein mit der Mischung aus Thrombin und Fibrinogen beaufschlagter Kollagenkörper zur Blutstillung auf die Wunde gepreßt.

In dieser Anwendung wird die zum Teil erhebliche Saugfähigkeit bzw. Aufnahmefähigkeit von Kollagenschwämmen als Unterstützung der Blutstillung ausgenutzt.

Dabei wird eine Komponente, z.B. Fibrinogen, zunächst auf den Schwamm aufgetragen und die zweite Komponente, nämlich Thrombin, erst unmittelbar vor der Applikation. Alternativ kann man auch die gemischten Komponenten kurz vor der blutstillenden Anwendung auf den Schwamm aufbringen.

Seit einigen Jahren werden gattungsgemäße Produkte auch in einer Kombination aus Kollagen und Fibrinogen sowie Thrombin als gebrauchsfertige Hämostatika angeboten. Die Blutproteine sind dabei verfahrenstechnisch so aufgebracht, daß die beschriebene Reaktion zunächst nicht zustande kommt und das Fertigprodukt im trockenen Zustand bei Raumtemperatur lagerfähig ist. Die Polymerisation der Fibrinkleber-Komponenten geschieht dabei erst unmittelbar durch Einwirken der Blutflüssigkeit während der blutstillenden Anwendung.

Aus der US 4 606 337 A und der EP 0 090 997 A2 sind gattungsgemäße Anordnungen bekannt, wobei die beiden Fraktionen, die einerseits Kollagen mit Fibrinogen und andererseits Kollagen mit Thrombin umfassen, jeweils als gesonderte Schicht vorliegen und zwei derartige Schichten miteinander verbunden sind.

Ein solcher Schichtaufbau weist den wesentlichen Nachteil auf, daß die Reaktionsfläche zwischen den beiden Fraktionen minimal ist und eine Reaktion sich ausgehend von der Grenzfläche allmählich, d. h. zeitlich vergleichsweise langsam, ausbreitet.

Hiervon ausgehend liegt der Erfindung die Aufgabe zugrunde, die bekannten Anordnungen so weiterzubilden, daß die Grenzfläche zwischen den Fraktionen wesentlich erhöht und dementsprechend optimiert wird und eine schnelle Reaktion, ausgehend von beliebigen Stellen des Produkts, möglich ist.

Diese Aufgabe wird erfindungsgemäß dadurch gelöst, dass das Kollagen in einer feinen pulverförmigen oder kleinteilig fibrillenartigen, lyophilisierten oder andersartig getrockneten Form vorliegt und die beiden Fraktionen gleichmäßig gemischt sind.

Eine erfindungsgemäße Mischung von pulvrigen Fraktionen schafft eine maximale gemeinsame Oberfläche, was in Verbindung mit Wundflüssigkeit zu einer optimalen schnellen Reaktion führt und den schnellen Verschluss von Wunden, wie z. B. Schusswunden, ermöglicht.

Vorzugsweise wird jede Fraktion dadurch hergestellt, daß eine Einmischung in das Substrat und eine anschließende Gefriertrocknung erfolgt oder die Fraktionen werden durch Benetzung mit den Blutproteinen und anschließender, die Struktur und Funktion erhaltende Trocknung hergestellt.

Mit Vorteil können neben den erwähnten Blutproteinen auch weitere Blutbestandteile humanen oder tierischen Ursprungs, wie Aprotinin zur Fibrinolysehemmung oder dergleichen, eingesetzt werden.

Die beiden erfindungsgemäßen Kollagenfraktionen werden günstigerweise im getrockneten Zustand gemischt und mittels eines spritzkolbenartigen Applikators appliziert.

Gemäß einer bevorzugten Ausführungsform zur Behandlung von Stichoder Schußwunden oder zur Hämostase von Gefäßinzissionen nach arteriellen Kathederapplikationen (bzw. deren Entfernung) können die Fraktionen in Form eines Zäpfchens oder Tampons gepreßt werden.

Bei der Applikation in den Stich- oder Schußkanal wird dann einerseits eine rein mechanische Verschlußwirkung, die durch die Quellung der Kollagenfasern bei Kontakt mit Körperflüssigkeiten verstärkt wird, und andererseits eine Stillung der Blutung einerseits durch die hämostatische Wirkung der Kollagenfasern andererseits durch die Polymerisation der Blutproteine (als Verklebung) erzielt.

Nachfolgend wird die Erfindung anhand eines Ausführungsbeispiels näher erläutert:

### Ausführungsbeispiel 1:

Zur Behandlung von frischen Schuß- und Stichverletzungen wird ein Kollagenimplantat entwickelt mit folgenden Eigenschaften:

Das Implantat ist aus einer Fraktion Kollagen mit Fibrinogen (3 bis 8 mg), sowie einer Fraktion Kollagen mit Thrombinzugabe in einer Dosierung von je 1 bis 4 I.E. In der Fraktion 2 ist daneben Aprotinin in einer Dosierung von 0,04 bis 0,1 Ph-Eur.E. eingearbeitet. Als Infektionsschutz enthält die erste Fraktion zusätzlich eine Dosis von 100 bis 200 mg Gentamicin.

Die beiden Kollagenfraktionen werden jeweils lyophilisiert und nach vollständiger Trocknung granuliert, anschließend zu gleichen Teilen vermischt und zu einem länglichen Zylinder von ca. 50 mm Länge und 8 mm Durchmesser verdichtet.

Das so gestaltete Implantat wird in einem Kunststoffapplikator verpackt, der geeignet ist, das Implantat in eine röhrenförmige Wunde (z.B. dem Wundkanal bei einer Schußverletzung) einzuführen und freizusetzen.

Bei Kontakt mit Blutflüssigkeit wird einerseits die Polymerisationsreaktion von Fibrinogen und Thrombin einsetzen und die Blutung zunehmend hemmen, andererseits wird eine Quellreaktion zusätzlich einen mechanischen Druck auf die verletzten Gefäße ausüben, so daß auch auf diese Weise eine temporäre Blutstillung unterstützt wird.

Der blutstillende Gegenstand ist also zur Erstversorgung von Verletzten gedacht. In einer dampfdichten Verpackung steril angereicht, kann er in entsprechenden Sanitätsausrüstungen Platz finden, da keine spezielle Tiefkühllagerung notwendig ist Die Erstversorgung wird durch die Freisetzung des Antibiotikums zusätzlich unterstützt.

### Ausführungsbeispiel 2:

Es wird ein Gegenstand zur blutstillenden Versorgung von Gefäßinzissionen nach Herzkatheteruntersuchungen beschrieben:

Der Gegenstand aus Kollagenpartikeln wird durch mechanisches Verpressen in eine zylindrische Form gebracht. Die eine Seite des Kollagenzylinders ist nach innen gewölbt und hat zur Aufgabe, eine Arterie eines bestimmten Umfanges bei Applikationen teilweise zu umfassen und die Wundränder der Gefäßwandöffnung zu adaptieren.

Die Einführung erfolgt über ein röhrenförmiges Instrument, in welches das Kollagen eingelagert wurde.

Der Grundaufbau des Kollagenzylinders entspricht dem aus dem Ausführungsbeispiel 1, wobei nicht der gesamte Kollagenzylinder aus der Vereinigung zweier Fraktionen aufgebaut ist, sondern nur der vordere Teil, der unmittelbar die Gefäßöffnung abdichten soll.

Der hintere Teil des Zylinders kann aus einem nicht weiter durch Zusatzproteine behandelten Kollagen bestehen und hat nur die Funktion, den mechanischen Druck auf die Spitze, d.h. dem der Gefäßöffnung nächsten Teils des Implantates, zu erhöhen.

Der durch das Aufnehmen von Wundflüssigkeit langsam nachlassende mechanische Stabilität und damit reduzierte Blutstillung durch Nachlassen der Andruckkraft wird durch die verklebende Wirkung des entstehenden Kollagen-Fibrin-Komplexes entgegengewirkt.

## Patentansprüche

1. Blutstillende Zubereitung umfassend die Komponenten Kollagen, Fibrinogen und Thrombin, wobei das Kollagen in zwei Fraktionen aufgeteilt ist und eine erste Fraktion mit einer Fibrinogenlösung behandelt und getrocknet und eine zweite Fraktion mit einer Thrombinlösung behandelt und getrocknet ist, **dadurch gekennzeichnet, daß** das Kollagen in einer feinen pulverförmigen oder kleinteilig fibrillenartigen, lyophilisierten oder andersartig getrockneten Form vorliegt und die beiden Fraktionen gleichmäßig gemischt sind.

2. Zubereitung nach Anspruch 1, **dadurch gekennzeichnet, daß** die Herstellung durch Einmischen in das Substrat und anschließende Gefriertrocknung oder durch Trocknung von benetzten Kollagenpartikeln bewerkstelligt ist.

3. Zubereitung nach Anspruch 1, **dadurch gekennzeichnet, daß** den Fraktionen weitere Blutbestandteile humaner oder tierischen Ursprungs, wie Aprotinin zur Fibrinolysehemmung oder dergleichen zugesetzt ist.

4. Zubereitung nach Anspruch 1, **dadurch gekennzeichnet, daß** die Fraktionen zu einem Formkörper, insbesondere in Form eines Zäpfchens oder Tampons, gepreßt sind.

## Claims

1. Anthemorrhagic preparation comprising the components collagen, fibrinogen and thrombin, wherein the collagen is divided into two fractions and a first fraction is treated with a fibrinogen solution and dried and a second friction is treated with a thrombin solution and dried, **characterized in that** the collagen is available in a fine powdery or small-pieced fibrillar-like, lyophilized or differently dried form and the two fractions are equally mixed.

2. Preparation according to claim 1, **characterized in that** the making is accomplished by incorporating the substrate and subsequent freeze drying or by drying dampened collagen particles.

3. Preparation according to claim 1, **characterized in that** further blood components of human or animal origin, like aprotinin for fibrinogenolysis inhibition or the like, are added to the fractions.

4. Preparation according to claim 1, **characterized in that** the fractions are pressed to form a molded body, in particular in form of a suppository or tampon.

## Revendications

1. Préparation hémostatique contenant les composés collagène, fibrinogène et thrombine, le collagène étant réparti en deux fractions, une première fraction étant traitée avec une solution de fibrinogène et séchée, et une deuxième fraction étant traitée avec une solution de thrombine et séchée, **caractérisée en ce que** le collagène est présent à l'état finement pulvérulent ou particulaire de type fibrilles, lyophilisé ou séché d'une autre manière, et **en ce que** les deux fractions sont mélangées de façon homogène.

2. Préparation selon la revendication 1, **caractérisée en ce que** la fabrication est effectuée par l'ajout d'un mélange au substrat, et par lyophilisation ou séchage subséquent de particules de collagène mouillées.

3. Préparation selon la revendication 1, **caractérisée en ce que** d'autres composés sanguins d'origine humaine ou animale, tels que de l'aprotinine, sont ajoutés aux fractions pour l'inhibition de la fibrinolyse ou analogue.

4. Préparation selon la revendication 1, **caractérisée en ce que** les fractions sont comprimées en un corps conformé, notamment sous la forme d'un suppositoire ou d'un tampon.
